# EUROPÄISCHE PATENTSCHRIFT

(11) **EP 1 560 564 B1**
(45) Veröffentlichungstag und Bekanntmachung des Hinweises auf die Patenterteilung: **02.01.2008**
(21) Anmeldenummer: 03773702.0
(22) Anmeldetag: 04.11.2003
(51) Int. Cl.: A61K 9/00

(54) **MEHRSCHICHTIGES TRANSMUCOSALES THERAPEUTISCHES SYSTEM**
MULTI-LAYER TRANSMUCOSAL THERAPEUTIC SYSTEM
SYSTEME THERAPEUTIQUE MULTICOUCHE D'ADMINISTRATION PAR VOIE TRANSMUQUEUSE

(30) Priorität: 13.11.2002 DE 10252726
(43) Veröffentlichungstag der Anmeldung: 10.08.2005
(73) Patentinhaber: LTS LOHMANN Therapie-Systeme AG, 56626 Andernach (DE)
(72) Erfinder: KRUMME, Markus, 56567 Neuwied (DE)
(74) Vertreter: Flaccus, Rolf-Dieter
(86) Internationale Anmeldenummer: PCT/EP2003/012272
(87) Internationale Veröffentlichungsnummer: WO 2004/043426

(56) Entgegenhaltungen:
- EP-A- 0 275 550
- WO-A-99/55312
- US-A- 4 615 697
- US-A- 4 772 470
- US-A- 4 855 142
- US-A- 4 876 092
- US-A1- 2002 142 036
- CUI ZHENGRONG ET AL: "Bilayer films for mucosal (genetic) immunization via the buccal route in rabbits" PHARMACEUTICAL RESEARCH (NEW YORK), Bd. 19, Nr. 7, Juli 2002 (2002-07), Seiten 947-953, XP009028363 ISSN: 0724-8741
- SOLOMONIDOU D ET AL: "Effect of carbomer concentration and degree of neutralization on the mucoadhesive properties of polymer films" JOURNAL OF BIOMATERIALS SCIENCE POLYMER EDITION, Bd. 12, Nr. 11, 2001, Seiten 1191-1205, XP009028488 ISSN: 0920-5063

## Beschreibung

Die Erfindung betrifft mehrschichtige filmförmige therapeutische Systeme zur transmucosalen Verabreichung von Wirkstoffen, insbesondere von Arzneistoffen. Diese Systeme sind geeignet, Wirkstoffe schnell und über einen längeren Zeitraum in kontrollierter Weise abzugeben.

Mucoadhäsive Arzneiformen sind im Stand der Technik bekannt, beispielsweise in Form von mucoadhäsiven Tabletten, Disks oder filmförmigen Darreichungsformen. Einige solcher Arzneiformen sind bereits auf dem Markt erhältlich. Mucoadhäsive Arzneiformen werden auf die Schleimhaut appliziert, insbesondere auf die Mundschleimhaut (buccale und/oder gingivale Schleimhaut), wodurch die Abgabe des darin enthaltenen Wirkstoffs und die Resorption über die Schleimhaut ermöglicht wird. Vorteilhaft ist dabei, daß die Wirkstoffe schnell in den Kreislauf gelangen und ein rascher Wirkungseintritt erzielt werden kann. Insbesondere eignen sich solche Arzneiformen zur Verabreichung von solchen Wirkstoffen, die aus dem Gastrointestinaltrakt nur schlecht resorbiert werden, und/oder die eine kurze Plasma-Halbwertszeit aufweisen.

Die bekanntesten mucoadhäsiven Arzneiformen sind Tabletten, die zweischichtig aufgebaut sind und aus einer mucoadhäsiven Schicht und einer retardierenden Rückschicht bestehen (Aftab^{®}, Fa. Rottapharm). Es wurden Anstrengungen unternommen, die Funktionsfähigkeit solcher mucoadhäsiver Tabletten zu verbessern, beispielsweise durch Anbringen von Drainage-Kerben, welche den Abtransport von Speichelflüssigkeit von der Applikationsstelle ermöglichen sollen. Solche Tablettensysteme sind zwar funktionsfähig, werden von den Patienten jedoch als unangenehm empfunden, da sie relativ dick, hart und unflexibel sind und dadurch ein deutliches Fremdkörpergefühl hervorrufen.

Daneben sind mucoadhäsive "Disks" bekannt, die auf der Basis von lipophilen, unlöslichen Polymermatrices und hydrophilen mucoadhäsiven Polymeren und gegebenenfalls Tensiden formuliert werden können. Diese Disks haben üblicherweise eine Dicke von ca. 1 mm und rufen deshalb im Mund eine unangenehme Fremdkörperempfindung hervor.

Aus US 4 713 243 sind ein- oder mehrschichtige mucoadhäsive Filme bekannt, deren mucoadhäsive Schicht aus Hydroxypropylcellulose, einem Ethylenoxid-Homopolymer, einem wasserunlöslichen Polymer (z. B. Ethylcellulose, Propylcellulose, Polyethylen, Polypropylen) und einem Weichmacher besteht. Diese Darreichungsformen werden von den Patienten zwar als etwas angenehmer empfunden, allerdings ist ihre Brauchbarkeit aufgrund der nur kurzen Klebezeit stark eingeschränkt. Die kurze Klebdauer ist dadurch bedingt, daß die verwendeten mucoadhäsiven Polymere leicht wasserlöslich sind, so daß eine nennenswerte Retardierung der Klebung nicht erfolgt. Um zu erreichen, daß die genannten mucoadhäsiven Filme über einen längeren Zeitraum auf der Schleimhaut anhaften, muß der Anteil der wasserunlöslichen PolymerKomponenten (z. B. Ethylcellulose, Propylcellulose) in der Formulierung erhöht werden. Dies hat allerdings zur Folge, daß die so hergestellten mucoadhäsiven Systeme eine größere Dicke aufweisen, und dadurch das Fremdkörpergefühl während der Applikationsdauer verstärkt wird. Zudem bringt die größere Dicke eine Abnahme der Wirkstofffreisetzung mit sich, da die Diffusionswege länger werden und die Diffusionskoeffizienten abnehmen.

Aus WO 99/55312 A2 ist ein mehrschichtiger, filmförmiger Arzneistoffträger mit einer mucoadhäsiven Schicht bekannt, die Polyvinylalkohol oder Polyacrylsäure enthalten kann. Dieser filmförmige Arzneimittelträger weist eine Rückschicht auf, die Hydroxypropylcellulose und Alkylcellulose enthält. Die Rückschicht soll nicht als Wirkstoffreservoir dienen

US 2002/142036 A1 beschreibt eine wirkstoffhaltige, mehrschichtige Folie, umfassend mucoadhäsive Schichten auf Basis von Polyacrylsäure und Methylcellulose.

Solomonidou, D. et al. beschreiben im Journal of Biomaterials Science Polymer Edition, Bd. 12, Nr. 11, 2001, Seiten 1191-1205, Filmformulierungen auf Basis von PVP oder PVA, bei denen Cellulose-Acetatbutyrat als Material für eine Rückschicht vorgesehen ist.

Es ist auch vorgeschlagen worden (US 5 719 197), die Kohärenz von mucoadhäsiven Systemen durch Verwendung von Ton als Zuschlagsstoff zu verbessern. Allerdings sind solche Tone wegen ihrer Eigenschaft, bestimmte Wirkstoffe zu adsorbieren oder durch katalytische Effekte die Wirkstoffstabilität zu beeinträchtigen, als nachteilig anzusehen. Außerdem wird durch diese Zuschlagstoffe das Gewicht und die Dicke des Systems deutlich erhöht.

Die der Erfindung zugrunde liegende Aufgabe bestand deshalb darin, mucoadhäsive Darreichungsformen bereitzustellen, welche die genannten Nachteile, insbesondere Hervorrufen eines Fremdkörpergefühls, unzureichende Wirkstofffreisetzung und zu kurze Klebdauer, nicht aufweisen.
Des weiteren sollen diese mucoadhäsiven Arzneiformen einerseits einen schnellen Wirkungseintritt und andererseits eine über einen längeren Zeitraum andauernde, kontinuierliche und kontrollierte Wirkstoffabgabe ermöglichen.

Die Aufgabe wird überraschenderweise gelöst durch filmförmige, mindestens zweischichtige transmucosale therapeutische System nach Anspruch 1 und die in den abhängigen Ansprüchen beschriebenen bevorzugten Ausführungsformen.

Gemäß Anspruch 1 sind die erfindungsgemäßen therapeutischen Systeme, die sich insbesondere zur transmucosalen Verabreichung von Wirkstoffen eignen, aus mindestens zwei miteinander verbundenen Schichten aufgebaut. Mindestens eine der Schichten ist wirkstoffhaltig. Eine der beiden Seiten des erfindungsgemäßen Systems wird durch eine mucoadhäsive Schicht begrenzt, die wahlweise wirkstoffhaltig oder wirkstofffrei ist. Diese mucoadhäsive Schicht befindet sich bei der Applikation in Kontakt mit der resorbierenden Schleimhaut, z. B. Mundschleimhaut. Die mucoadhäsive Schicht des Systems ist mit einer Rückschicht verbunden, welche einschichtig oder mehrschichtig ist und als Wirkstoffreservoir dient. Eine besondere Eigenschaft der mucoadhäsiven Schicht besteht darin, daß diese in wässrigen Medien quellbar, jedoch unlöslich oder nur schwer, d. h. langsam, löslich ist. Durch die Unlöslichkeit oder verminderte Löslichkeit wird die Klebezeit auf der Schleimhaut erhöht und dadurch eine Wirkstofffreisetzung über einen längeren Zeitraum ermöglicht. Da die erfindungsgemäßen Systeme filmförmig sind und vorzugsweise eine Dicke von weniger als 1 mm aufweisen, verursachen sie kein Fremdkörpergefühl und werden von den Patienten nicht als unangenehm empfunden, wodurch die Akzeptanz dieser Arzneiformen verbessert wird.

Als "wässrige Medien" werden neben Wasser insbesondere physiologische Flüssigkeiten, insbesondere Speichel, verstanden.

Die mucoadhäsive Schicht besteht im wesentlichen aus einem Polymergemisch, welches filmbildend, in wässrigen Medien quellbar, jedoch darin nicht oder nur schwer löslich ist. Das Polymergemisch umfaßt mindestens ein hydrophiles, mucoadhäsives Polymer, das in einer Polymermatrix eingebettet oder dispergiert ist. Optional kann die mucoadhäsive Schicht Wirkstoff(e) oder Zusatzstoffe enthalten.

Das genannte hydrophile Polymer bzw. die hydrophilen Polymere ist/sind bevorzugt aus der Gruppe ausgewählt, die carboxylgruppentragende hydrophile Kleberpolymere, Polyacrylate oder Polyacrylsäurederivate (z. B. Carbopol^{®}-Typen, Fa. B. F. Goodrich) und deren Salze, Carboxymethylcellulose und deren Salze, Poly(methylvinylether-maleinsäureanhydrid) und deren wässrige oder alkoholische Hydrolysate und Salze (z. B. Gantrez^{®}-Typen, wie Gantrez-AN, -S, -ES, -MS; Fa. ISP) umfaßt.

Die genannte Polymermatrix der mucoadhäsiven Schicht basiert im wesentlichen auf hydrophilen, aber in wässrigen Medien unlöslichen oder langsam löslichen Polymeren. Dieses Polymer bzw. die Polymere ist/sind aus der Gruppe der Polyvinylalkohole ausgewählt.
Auch andere dem Fachmann bekannte Polymere, welche eine in trockenem Zustand oder in wässriger Umgebung dauerhafte Verankerung der mucoadhäsiven Schicht auf der benachbarten Rückschicht ermöglichen, können verwendet werden.

Um eine dauerhafte Verbindung zwischen der mucoadhäsiven Schicht und der darüber liegenden Rückschicht (bzw. einer der einzelnen Schichten der Rückschicht, im Falle einer mehrschichtigen Rückschicht) zu gewährleisten, ist es vorteilhaft, für die Herstellung der Polymermatrix der mucoadhäsiven Schicht solche Basispolymere auszuwählen, die mit den für die Herstellung der Rückschicht verwendeten Polymere identisch sind oder zumindest chemisch verwandt sind. Allgemein wird bevorzugt, daß benachbarte Schichten des filmförmigen Systems ein oder mehrere identische oder chemisch verwandte Basispolymere enthalten, welche vorzugsweise aus der Gruppe der Polyacrylate ausgewählt sind. Auf diese Weise kann sichergestellt werden, daß die mucoadhäsive Schicht auch in wässriger Umgebung (Mundhöhle) während des Applikationszeitraums mit der Rückschicht dauerhaft verbunden bleibt.

Gemäß einer bevorzugten Ausführungsform werden die Polymere der mucoadhäsiven Schicht mittels physikalischer oder/und chemischer Methoden vernetzt. Durch die Vernetzung kann der Grad der Löslichkeit reduziert werden, ohne daß die Hydrophilie beeinträchtigt wird. Auf diese weise kann die Klebedauer des Systems auf der Schleimhaut zusätzlich und besonders vorteilhaft verbessert werden. Geeignete Vernetzungsreagenzien und -verfahren sind dem Fachmann bekannt (z. B. Verwendung von Aluminiumacetylacetonat oder Titanylacetylacetonat als vernetzendes Agens).

Die mucoadhäsive Schicht kann zur Modulation der Klebeigenschaften geeignete Zusatzstoffe enthalten, welche dem Fachmann bekannt sind.

Die Rückschicht oder (im Falle einer mehrschichtigen Rückschicht) die einzelnen Schichten der Rückschicht wird / werden auf der Basis von Polyacrylaten hergestellt, insbesondere auf der Basis von neutralisierten Polymethylmethacrylaten (z. B. Eudragit^{®} E 100, Eudragit^{®} NE 30 D, Plastoid^{®} B; Röhm Pharma). Besonders bevorzugt sind dabei Polyacrylate, welche in wässrigen Medien - weitgehend pH-unabhängig - quellbar, jedoch nicht löslich sind.
Die Rückschicht oder mindestens eine der die Rückschicht bildenden Schichten kann optional einen oder mehrere Hilfsstoffe enthalten, vorzugsweise ausgewählt aus der Gruppe der Weichmacher, Penetrations-Enhancer, Lösungsvermittler, Farbstoffe, Pigmente und Matrixbildner. Hierfür geeignete Stoffe sind dem Fachmann bekannt.

Als Weichmacher kommen beispielsweise solche aus der Gruppe der Kohlenwasserstoffe, Alkohole (insbesondere höhere Alkohole wie Dodecanol, Undecanol, Octanol), mehrwertige Alkohole, Polyethylenglykole, Triglyceride, Carbonsäuren, Derivate von Carbonsäuren, Ether, Ester (z. B. Diethylphthalat, n-Butyladipat, Citronensäure-Ester) und Amine in Betracht.

Als Resorptions- oder Permeationsbeschleuniger (Enhancer) eignen sich insbesondere Stoffe, die ausgewählt sind aus der Gruppe, die folgende Stoffe bzw. Stoffklassen umfaßt: gesättigte oder ungesättigte Fettsäuren, Fettsäure-Ester, insbesondere Ester mit Methanol, Ethanol oder Isopropanol (z. B. Ölsäureethylester, Ölsäuremethylester, Laurinsäuremethylester, Laurinsäureethylester, Adipinsäuremethylester, Adipinsäureethylester), geradkettige oder verzweigte Fettalkohole bzw. deren Ester, insbesondere Ester mit Essigsäure oder Milchsäure (z. B. Ethyloleat, Ethyllaurat, Ethylpalmitat, Ethyllactat, Propyllactat, Propylpalmitat, Propyllaurat, Propyloleat), mehrwertige aliphatische Alkohole oder Polyethylenglykole, Sorbitanfettsäureester und deren durch Ethoxylierung erhältlichen Derivate, Fettalkoholethoxylate, Polyoxyethylenfettsäureester; Laurinsäurediethanolamid, Ölsäurediethanolamid, Kokosfettsäurediethanolamid, D-alpha-Tocopherol, Laurinsäurehexylester, 2-Octyldodecanol, Dexpanthenol, Isopropylidenglycerol, Transcutol (= Diethylenglycol-monoethylether), DEET (= N,N-Diethyl-m-Toluolamid), Solketal, Ethanol, 1,2-Propandiol oder andere kurzkettige Alkohole (d. h. Alkohole mit bis zu 6 C-Atomen), sowie Menthol und andere ätherische Öle oder Bestandteile ätherischer Öle. Um den Wirkstoff-Flux zu optimieren, können auch Kombinationen zweier oder mehrerer Enhancer eingesetzt werden.

Der gesamte Mengenanteil von Weichmachern und permeationsverbessernden Substanzen kann bis ca. 10 Gew.-% betragen, bezogen auf die filmförmige Arzneiform. Besonders bevorzugt ist ein Gehalt von weniger als 5 Gew.-%, insbesondere von weniger als 1 Gew.-%.

Als Beispiele für Lösungsvermittler seien mehrwertige Alkohole wie 1,2-Propandiol, Butandiole, Glycerin, Polyethylenglycol 400, Tetrahydrofurfurylalkohol, Diethylenglykolmonoethylether, Diethyltoluamid und Monoisopropylidenglycerin genannt. Der Anteil des/der Lösungsvermittlers, bezogen auf eine Arzneiform, kann zwischen 0,1 und 10 Gew.- % betragen, vorzugsweise 0,5 bis 5 Gew.-%.

Als Pigmente kommen insbesondere Talkum, Titandioxid, Eisenoxide oder plättchenförmige Pigmente in Betracht. Der Pigmentanteil kann bis zu 80, vorzugsweise bis zu 70 Gew.-% betragen, bezogen auf den Polymer-Anteil in der jeweiligen Schicht.

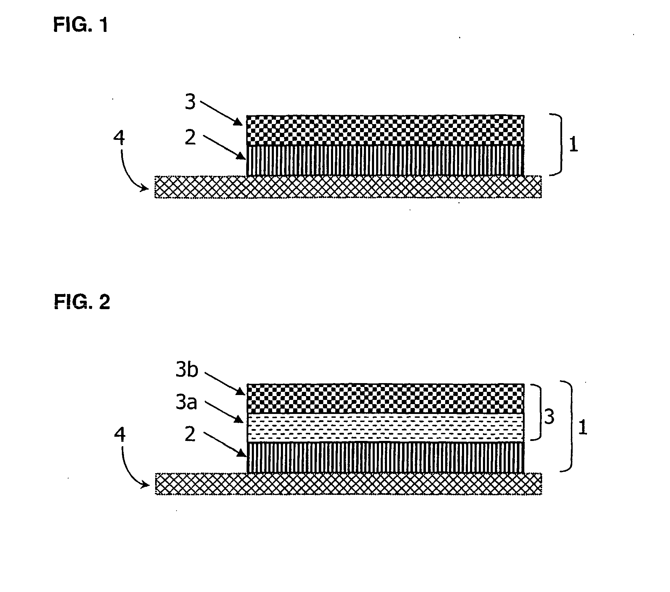

Die erfindungsgemäßen filmförmigen mucoadhäsiven Systeme sind im einfachsten Fall aus zwei Schichten aufgebaut, einer mucoadhäsiven Schicht und einer damit verbundenen Rückschicht, die als Wirkstoffreservoir dient (Fig. 1). Zusätzlich kann auch die mucoadhäsive Schicht wirkstoffhaltig sein und vorzugsweise denselben Wirkstoff enthalten, der auch in der Rückschicht enthalten ist.
Die Wirkstoffabgabe aus dem System an die Schleimhaut erfolgt durch Diffusion aus den Schichten des Systems.

Gemäß einer bevorzugten Ausführungsform ist vorgesehen, daß die Rückschicht durch geeignete Zuschlagstoffe derart modifiziert ist, daß die Permeation von Wasser und die Diffusion von Wirkstoff in dieser Schicht herabgesetzt oder blokkiert ist, relativ zu der Diffusion und Permeation in der mucoadhäsiven Schicht.

In weiteren Ausführungsformen der Erfindung ist vorgesehen, daß die Systeme als Multilayer gestaltet sind und vorzugsweise bis zu 6 einzelne Schichten umfassen, wobei eine Schichten-Anzahl von 2 bis 4 besonders bevorzugt ist. Jeweils eine der Oberflächen des Systems wird dabei von einer mucoadhäsiven Schicht gebildet. Vorzugsweise enthalten sämtliche Schichten denselben Wirkstoff, in gleichen oder unterschiedlichen Konzentrationen.

Der mehrschichtige Aufbau ermöglicht die Herstellung von erfindungsgemäßen Systemen, welche direkt nach der Applikation eine initiale Burst-Dosis freisetzen und nachfolgend mit verringerter Abgaberate eine Erhaltungsdosis über einen längeren Zeitraum (mehrere Stunden, vorzugsweise 0,5 bis 24 h) freisetzen.

Besonders vorteilhaft sind Ausführungsformen, bei denen die Rückschicht aus zwei oder mehreren übereinander geschichteten und miteinander verbundenen Einzelschichten aufgebaut ist. Auf diese weise kann die im System enthaltene Wirkstoffdosis erhöht werden. Zudem können die einzelnen Schichten Zuschlagstoffe enthalten, durch welche die Löslichkeit und der Diffusionskoeffizient des Wirkstoffs in der jeweiligen Schicht modifiziert wird. Dadurch erhält man ein Multilayer mit einem definierten Konzentrationsgradienten. Diese Ausführungsform ist besonders vorteilhaft. Die Ausbildung eines Konzentrationsgradienten kann zusätzlich dadurch unterstützt werden, daß der Wirkstoff in den einzelnen Schichten in auf- bzw. absteigenden Mengen oder Konzentrationen enthalten ist.

Gemäß einer weiteren bevorzugten Ausführungsform ist vorgesehen, daß die Rückschicht oder diejenige äußere Schicht, welche sich auf der der mucoadhäsiven Oberfläche gegenüberliegenden Seite des Systems befindet und dort die äußere Oberfläche bildet, durch geeignete Zuschlagstoffe derart modifiziert ist, daß die Permeation von Wasser und die Diffusion von Wirkstoff in dieser Schicht herabgesetzt oder blockiert ist, relativ zu der Diffusion und Permeation in der mucoadhäsiven Schicht oder in den anderen Schichten der Rückschicht.

Auf diese Weise wird ein transmucosales System erhalten, das einen mindestens dreischichtigen Aufbau aufweist, mit einer mucoadhäsiven Schicht, mindestens einer damit verbunden mittleren Reservoir-Schicht, und einer damit verbundenen äußeren Schicht oder Grenzschicht (Fig. 2). In der letztgenannten Schicht ist die Wirkstoffdiffusion - relativ zu der/den mittleren Schicht(en) - vermindert oder sogar vollständig blockiert.

Die Modifizierung der Diffusions- und Permeationseigenschaften kann insbesondere durch Variation des Pigmentanteils oder/und durch Beimischung geeigneter diffusionsverzögernder polymerer (z. B. Ethylcellulose, Propylcellulose) oder nicht-polymerer Hilfsstoffe bewirkt werden. Auf diese Weise können die Diffusionseigenschaften der Rückschicht bzw. der äußersten Schicht der Rückschicht zwischen zwei Extremen eingestellt werden, nämlich zwischen vollständiger Diffusionsblockade einerseits und praktisch ungehinderter oder unmodifizierter Wirkstoffdiffusion aus der Matrix andererseits. Somit können wahlweise Systeme hergestellt werden, die den/die Wirkstoff(e) einseitig freisetzen (d. h. nur auf der mucoadhäsiven Seite) oder zweiseitig freisetzen (d. h. auf der mucoadhäsiven Seite und auf der ihr gegenüberliegenden Seite des Systems).

Zumindest die mittlere(n) Schicht(en) des Systems ist/sind wirkstoffhaltig; vorzugsweise enthält auch die genannte äußere Grenzschicht denselben Wirkstoff bzw. dieselben Wirkstoffe. Ebenso kann auch die mucoadhäsive Schicht wirkstoffhaltig sein.

Durch den vorstehend beschriebenen, mindestens dreischichtigen Aufbau mit der genannten äußeren Schicht erhält man ein System, bei dem die Wirkstoffabgabe durch eine Kombination einer matrixgesteuerten Diffusionskontrolle und einer membrangesteuerten Freisetzung gesteuert wird.
Generell würde ein solches System, welches auf einer gemischten Steuerung (Kombination von Matrix- und Membransteuerung) beruht, den Wirkstoff in einer Art Sättigungsfunktion freisetzen, d. h. die Abgaberate des Systems würde mit zunehmender Ausnutzung des Systems immer weiter abnehmen. Durch geeignete Formulierung, insbesondere durch geeignete Wahl des/der Matrix-Polymere (Erhöhung des Anteils hydrophiler funktioneller Gruppen) oder durch Zusatz geeigneter hydrophiler, wasserbindender Zusatzstoffe (insbesondere Polyalkohole oder polymere Tenside mit hohem HLB-Wert, vorzugsweise HLB ≥ 10, insbesondere HLB ≥ 15), kann mit zunehmender Verweildauer des Systems im feuchten Medium (d. h. an der Applikationsstelle in der Mundhöhle) der Grad der Wasseraufnahme oder der Grad der Quellung der Reservoir-Matrix beeinflußt und erhöht werden.

Mittels der beschriebenen Maßnahme kann der Diffusionskoeffizient in der Reservoirschicht durch zunehmende Quellung oder durch Zunahme des Hydratisierungsgrades erhöht werden. Dies ermöglicht es, die durch die Abnahme des Konzentrationsgradienten verursachte Abnahme der Freisetzungsrate durch eine zunehmende Quellung und Hydratisierung der Wirkstoffmatrix derart zu kompensieren, daß dadurch eine im wesentlichen lineare Freisetzung resultiert, verbunden mit einer hohen Ausnutzung des Systems.

Diese Eigenschaften der erfindungsgemäßen Systeme sind insbesondere im Hinblick auf eine längerdauernde Applikation des Systems, beispielsweise über einen Zeitraum von mehreren Stunden (z. B. 2 bis 24 h), von Bedeutung. Dies gilt insbesondere dann, wenn die zu verabreichenden Stoffe ein entsprechend enges therapeutisches Fenster aufweisen.

Mindestens eine der Schichten der erfindungsgemäßen filmförmigen Systeme enthält einen Wirkstoff oder eine Wirkstoffkombination. Die Polymere der einzelnen Schichten bilden eine Polymermatrix, die als Wirkstoffreservoir dient. In dieser Polymermatrix ist/sind jeweils der/die Wirkstoff(e) in gelöster, suspendierter oder emulgierter Form enthalten, bevorzugt "gelöst" im Sinne einer "festen Lösung". Als Wirkstoffe kommen insbesondere Arzneistoffe in Betracht, besonders bevorzugt hochwirksame Arzneistoffe, z. B. aus folgenden Gruppen: auf das Nervensystem wirkende Stoffe, Psychopharmaka, Sedativa, Narkotika, Hormone, insulinartige Wirkstoffe, Analgetika, Antikonvulsiva, Antiparkinsonmittel, auf das kardiovaskuläre System wirkende Arzneistoffe, Antiinfektiva, Wirkstoffe zur Behandlung von Stoffwechselstörungen (z. B. Lipidsenker), auf das Muskelsystem wirkende Stoffe, und andere. Die erfindungsgemäßen Systeme eignen sich vor allem für die Verabfolgung solcher Arzneistoffe, die einer schnellen Metabolisierung unterliegen oder/und die auf gastrointestinalem Wege nur ungenügend resorbiert werden.
Die Erfindung wird nachfolgend anhand von Beispielen und Zeichnungen näher erläutert.

Fig. 1 und Fig. 2 zeigen in schematischer SchnittDarstellung den Schichtaufbau zweier beispielhafter erfindungsgemäßer mucoadhäsiver Systeme (1).

Fig. 1 zeigt ein System (1) mit einem zweischichtigen Aufbau, umfassend eine mucoadhäsive Schicht oder Kleberschicht (2) und eine damit verbundene Rückschicht oder Reservoir-schicht (3). Die mucoadhäsive Schicht (2) des abgebildeten Systems (1) befindet sich in klebendem Kontakt mit einer Schleimhaut (4); Zustand während der Applikation.

Fig. 2 zeigt ein System (1) mit einem dreischichtigen Aufbau, umfassend eine mucoadhäsive Schicht (2) und eine Rückschicht (3), die aus zwei einzelnen Reservoir-Schichten (3a, 3b) besteht, einer mittleren Schicht (3a) und einer äußeren Schicht oder Grenzschicht (3b). Die das System nach außen abschließende Reservoir-Schicht (3b) ist vorzugsweise diffusionskontrolliert gestaltet.

### Beispiel

### Herstellung eines dreischichtigen Systems (wie in Fig. 2)

Ein Wirkstoff wird in einem neutralen Polyacrylat (z. B. Eudragit^{®} NE 30 D; Fa. Röhm) entweder direkt gelöst, oder unter Verwendung eines geeigneten, dem Fachmann bekannten Lösungsmittel, erforderlichenfalls unter Verwendung eines Lösungsvermittlers oder Solubilisators. Die Wahl der geeigneten Methode hängt von der Löslichkeit bzw. den Lösungseigenschaften des verwendeten Wirkstoffs ab.
Ferner wird der wirkstoffhaltigen Polymerlösung ein geeignetes Pigment zugesetzt, z. B. Talkum, TiO₂, Eisenoxide oder plättchenförmige Pigmente, und eine homogene Flüssigkeit hergestellt. Der Pigmentanteil ist relativ hoch und liegt bei ca. 60 Gew.-%, bezogen auf den Polymer-Anteil. Anschließend wird die Viskosität der Flüssigkeit so eingestellt, daß sie für die nachfolgenden Verarbeitungsmethoden geeignet ist. Die Flüssigkeit wird vorzugsweise mittels eines Gießverfahrens oder Sprühverfahrens auf eine inerte Unterlage aufgetragen und einer anschließenden Trocknung unterworfen, woraus ein dünner Film resultiert. Die verwendete inerte Unterlage muß so beschaffen sein, daß der Film nach der Trocknung an ihr haften bleibt, jedoch ohne Zerstörung von der Unterlage abgelöst werden kann.

Auf dieselbe Weise wie vorstehend beschrieben wird eine zweite Flüssigkeit hergestellt, die sich von der ersten Formulierung nur durch das Fehlen des Pigments oder durch einen niedrigeren Pigmentanteil unterscheidet. Dadurch ist der Wirkstoff-Anteil relativ zum Gesamt-Feststoffanteil erhöht, im Vergleich zu der zuerst hergestellten Flüssigkeit. Die zweite Flüssigkeit wird wiederum mittels eines Sprüh-oder Gießverfahrens auf die zuerst hergestellte Schicht beschichtet und nachfolgend getrocknet, so daß ein Zweischichtlaminat mit zwei Reservoir-Schichten erhalten wird.

Zur Erzeugung der mucoadhäsiven Schicht wird eine wässrige Lösung von hochhydrolysiertem Polyvinylalkohol (z. B: Mowiol 28-99, Fa. Clariant) geeigneter Konzentration (z. B. 10 Gew.-%; wahlweise 0,5 bis 60 Gew.-%) hergestellt und darin ein Kleberpolymer (z. B. Gantrez S 95, Fa. ISP) in einem geeigneten Anteil gelöst. Der Anteil des Kleberpolymers entspricht in diesem Beispiel dem Polyvinylalkohol-Anteil (d. h. Mischungsverhältnis 1:1; Gewichtsanteile). Es können aber auch andere Mischungsverhältnisse verwendet werden, z. B. im Bereich von 50:1 bis 1:50, bezogen auf Kleberanteil : Polyvinylalkohol-Anteil).
Die erhaltene, homogene Lösung wird wiederum mit einem geeigneten Auftragsverfahren auf das zuvor hergestellte Zweischicht-Laminat beschichtet und anschließend getrocknet. Dadurch erhält man ein - je nach Auftragsgewicht - ca. 50 bis 250 µm dickes Dreischichtlaminat. Die Oberseite dieses Laminats ist im feuchten Zustand gut klebrig und hat mucoadhäsive Eigenschaften. Das Laminat hat eine insgesamt sehr gute Flexibilität und bleibt nach der Applikation auf eine Schleimhaut über mehrere Stunden dort haften.

Die erfindungsgemäßen transmucosalen Systeme eigenen sich in vorteilhafter Weise zur Verabreichung von Wirkstoffen, insbesondere Arzneistoffen, zur therapeutischen oder prophylaktischen Behandlung in der Human- oder Tiermedizin.

## Patentansprüche

1. Filmförmiges, mindestens zwei miteinander verbundene Schichten umfassendes therapeutisches System (1) zur transmucosalen Verabreichung von Wirkstoffen, welches eine in wässriger Umgebung mucoadhäsive Schicht (2) sowie eine ein- oder mehrschichtige, auf der Basis von Polyacrylaten hergestellte Rückschicht (3) aufweist, die als Wirkstoffreservoir dient, und wobei mindestens eine der Schichten des Systems wirkstoffhaltig ist, und die genannte mucoadhäsive Schicht (2) in wässrigen Medien quellbar, jedoch unlöslich oder nur schwer löslich ist und ein Polymergemisch enthält, welches mindestens ein hydrophiles, mucoadhäsives Polymer umfaßt, das in einer auf Basis von Polyvinylalkohol(en) hergetellten Polymermatrix eingebettet oder dispergiert ist.

2. Filmförmiges therapeutisches System nach Anspruch 1, **dadurch gekennzeichnet, daß** die mucoadhäsive Schicht (2) im wesentlichen aus einem Polymergemisch besteht, welches filmbildend, in wässrigen Medien quellbar, jedoch darin nicht oder nur schwer löslich ist.

3. Filmförmiges therapeutisches System nach Anspruch 2, **dadurch gekennzeichnet, daß** das/die genannte(n) mucoadhäsive(n) Polymer(e) aus der Gruppe ausgewählt ist/sind, die carboxylgruppentragende hydrophile Kleberpolymere, Polyacrylate und deren Salze, Carboxymethylcellulose und deren Salze, Poly(methylvinylether-maleinsäureanhydrid) und deren wässrige oder alkoholische Hydrolysate und Salze umfaßt.

4. Filmförmiges therapeutisches System nach einem der Ansprüche 1 bis 3, **dadurch gekennzeichnet, daß** die Polymermatrix der mucoadhäsiven Schicht mittels physikalischer oder chemischer Methoden vernetzt worden ist.

5. Filmförmiges therapeutisches System nach einem der vorangehenden Ansprüche, **dadurch gekennzeichnet, daß** die Rückschicht (3) oder - im Falle einer mehrschichtigen Rückschicht - die einzelnen Schichten (3a, 3b) der Rückschicht auf der Basis von neutralisierten Polymethylmethacrylaten hergestellt ist/sind.

6. Filmförmiges therapeutisches System nach einem der vorangehenden Ansprüche, **dadurch gekennzeichnet, daß** die Rückschicht, oder mindestens eine der einzelnen Schichten der Rückschicht, einen oder mehrere Hilfsstoffe enthält, vorzugsweise ausgewählt aus der Gruppe der Weichmacher, Penetrations-Enhancer, Lösungsvermittler, Farbstoffe, Pigmente und Matrixbildner.

7. Filmförmiges therapeutisches System nach einem der vorangehenden Ansprüche, **dadurch gekennzeichnet, daß** benachbarte Schichten ein oder mehrere identische oder chemisch verwandte Basispolymere enthalten, vorzugsweise aus der Gruppe der Polyacrylate.

8. Filmförmiges therapeutisches System nach einem der vorangehenden Ansprüche, **dadurch gekennzeichnet, daß** es aus 2 bis 6 Schichten aufgebaut ist, vorzugsweise aus 2 bis 4 Schichten.

9. Filmförmiges therapeutisches System nach einem der vorangehenden Ansprüche, **dadurch gekennzeichnet, daß** die Rückschicht oder diejenige äußere Schicht, welche der mucoadhäsiven Seite gegenüberliegt, eine Grenzschicht bildet, in der die Permeation von Wasser und die Diffusion von Wirkstoff herabgesetzt ist, relativ zu der/den übrigen Schicht(en) des Systems.

10. Filmförmiges therapeutisches System nach einem der vorangehenden Ansprüche, **dadurch gekennzeichnet, daß** es mindestens dreischichtig aufgebaut ist und eine mucoadhäsive Schicht (2), mindestens eine mittlere Reservoir-Schicht (3a) und eine äußere Rückschicht (3b) umfaßt, wobei die letztgenannte Schicht eine Grenzschicht bildet, in der die Permeation von Wasser und die Diffusion von Wirkstoff herabgesetzt ist, relativ zu der/den übrigen Schicht(en) des Systems.

11. Filmförmiges therapeutisches System nach Anspruch 9 oder 10, **dadurch gekennzeichnet, daß** die genannte Grenzschicht Zuschlagstoffe enthält, welche die Diffusion des Wirkstoffs herabsetzen oder blockieren, insbesondere Zuschlagstoffe aus der Gruppe der Pigmente und der diffusionsverzögernden Polymere.

12. Filmförmiges therapeutisches System nach einem der Ansprüche 9 bis 11, **dadurch gekennzeichnet, daß** die Reservoirschicht(en) einen oder mehrere Zusatzstoffe enthält/enthalten, welche die Quellfähigkeit und Hydratisierung der Reservoir-Matrix erhöhen, wobei diese Zusatzstoffe vorzugsweise aus der Gruppe der hydrophilen, wasserbindenden Stoffe ausgewählt sind, wobei Polyalkohole und polymere Tenside mit einem HLB-Wert ≥ 10 besonders bevorzugt werden.

13. Filmförmiges therapeutisches System nach einem der vorangehenden Ansprüche, **dadurch gekennzeichnet, daß** der/die Wirkstoff(e) in gelöster, suspendierter oder emulgierter Form vorliegen.

14. Filmförmiges therapeutisches System nach einem der vorangehenden Ansprüche, **dadurch gekennzeichnet, daß** zwei oder mehrere Schichten denselben Wirkstoff enthalten, vorzugsweise in unterschiedlichen Konzentrationen unter Ausbildung eines Konzentrationsgradienten.

15. Filmförmiges therapeutisches System nach Anspruch 14, **dadurch gekennzeichnet, daß** die einzelnen Schichten Zuschlagstoffe enthalten, durch welche die Löslichkeit und der Diffusionskoeffizient des Wirkstoffs in der jeweiligen Schicht modifiziert wird.

## Claims

1. Film-shaped therapeutic system (1) comprising at least two layers connected with each other, for transmucosal administration of active substances, which system (1) has a layer (2) which is mucoadhesive in aqueous environment and a mono-layered or multi-layered backing layer (3) prepared on the basis of polyacrylates and serving as active substance reservoir, and wherein at least one of the layers of said system contains active substance, and wherein the said mucoadhesive layer (2) is capable of swelling in aqueous media but is insoluble, or only poorly soluble, therein and contains a polymer mixture comprising at least one hydrophile, mucoadhesive polymer which is embedded or dispersed in a polymer matrix prepared on the basis of polyvinyl alcohol(s).

2. Film-shaped therapeutic system according to claim 1, **characterized in that** the mucoadhesive layer (2) is substantially made up of a polymer mixture which is film-forming, capable of swelling in aqueous media but insoluble, or only poorly soluble, therein.

3. Film-shaped therapeutic system according to claim 2, **characterized in that** the said mucoadhesive polymer(s) is/are selected from the group comprising carboxyl groups-carrying hydrophile adhesive polymers, polyacrylates and their salts, carboxymethyl cellulose and its salts, poly(methyl vinyl ether maleic anhydride) and its aqueous or alcoholic hydrolysates and salts.

4. Film-shaped therapeutic system according to any one of claims 1 to 3, **characterized in that** the polymer matrix of the mucoadhesive layer has been crosslinked by means of physical or chemical methods.

5. Film-shaped therapeutic system according to any one of the preceding claims, **characterized in that** the backing layer (3) or - in the case of a multi-layered backing layer - the individual layers (3a, 3b) of the backing layer is/are manufactured on the basis of neutralised polymethyl methacrylates.

6. Film-shaped therapeutic system according to any one of the preceding claims, **characterized in that** the backing layer, or at least one of the individual layers of the backing layer, contains one or more auxiliary substances, preferably selected from the group of the plasticizers, penetration enhancers, solubilizers, dyes, pigments and matrix formers.

7. Film-shaped therapeutic system according to any one of the preceding claims, **characterized in that** adjacent layers contain one or more identical or chemically allied base polymers, preferably from the group of the polyacrylates.

8. Film-shaped therapeutic system according to any one of the preceding claims, **characterized in that** it is made up of 2 to 6 layers, preferably of 2 to 4 layers.

9. Film-shaped therapeutic system according to any one of the preceding claims, **characterized in that** the backing layer, or that outer layer which is opposite to the mucoadhesive side, forms a boundary layer in which the permeation of water and the diffusion of active substance is reduced, relative to the other layer(s) of the system.

10. Film-shaped therapeutic system according to any one of the preceding claims, **characterized in that** it is made up of at least three layers and comprises a mucoadhesive layer (2), at least one middle reservoir layer (3a) and an outer backing layer (3b), with the latter layer forming a boundary layer in which the permeation of water and the diffusion of active substance is reduced, relative to the other layer(s) of the system.

11. Film-shaped therapeutic system according to claim 9 or 10, **characterized in that** the said boundary layer contains additives which reduce or block the diffusion of the active substance, particularly additives from the group of the pigments and the diffusion-retarding polymers.

12. Film-shaped therapeutic system according to any one of claims 9 to 11, **characterized in that** the reservoir layer(s) contain(s) one or more additives which increase the swelling capacity and the hydration of the reservoir matrix, said additives preferably being selected from the group of hydrophile, water-binding substances, with poly-acohols and polymeric surfactants with an HLB value of ≥ 10 being particularly preferred.

13. Film-shaped therapeutic system according to any one of the preceding claims, **characterized in that** the active substance(s) is/are present in dissolved, suspended or emulsified form.

14. Film-shaped therapeutic system according to any one of the preceding claims, **characterized in that** two or more layers contain the same active substance, preferably at different concentrations under formation of a concentration gradient.

15. Film-shaped therapeutic system according to claim 14, **characterized in that** the individual layers contain additives which cause a modification of the solubility and of the diffusion coefficient of the active substance in the respective layer.

## Revendications

1. Système thérapeutique (1) en forme de film, comprenant au moins deux couches assemblées ensemble pour l'administration transmuqueuse de substances actives, qui présente une couche muco-adhésive (2) dans un environnement aqueux ainsi qu'une couche dorsale à une ou plusieurs couches, réalisée à base de polyacrylates qui sert de réservoir de substances actives et au moins une des couches du système contenant une ou plusieurs substances actives et ladite couche muco-adhésive (2) étant gonflable, mais insoluble ou seulement peu soluble dans les milieux aqueux, et contenant un mélange de polymères qui comprend au moins un polymère hydrophile, muco-adhésif qui est incorporé ou dispersé dans une matrice polymère réalisée à base de poly(alcool(s) vinylique(s)).

2. Système thérapeutique en forme de film selon la revendication 1, **caractérisé en ce que** la couche muco-adhésive (2) est essentiellement constituée d'un mélange de polymères qui forme un film, qui est gonflable mais insoluble ou seulement peu soluble dans les milieux aqueux.

3. Système thérapeutique en forme de film selon la revendication 2, **caractérisé en ce que** le/les polymère(s) muco-adhésif(s) mentionné(s) est/sont choisi(s) dans le groupe comprenant les polymères adhésifs hydrophiles portant des groupes carboxyle, les polyacrylates et leurs sels, la carboxyméthylcellulose et ses sels, le poly(méthylvinyléther-anhydride de l'acide maléique) et ses hydrolysats aqueux ou alcooliques et ses sels.

4. Système thérapeutique en forme de film selon l'une quelconque des revendications 1 à 3, **caractérisé en ce que** la matrice polymère de la couche muco-adhésive a été réticulée au moyen de procédés physiques ou chimiques.

5. Système thérapeutique en forme de film selon l'une quelconque des revendications précédentes, **caractérisé en ce que** la couche dorsale (3) ou - dans le cas d'une couche dorsale à plusieurs couches - les différentes couches (3a, 3b) de la couche dorsale est/sont réalisée(s) à base de poly(méthacrylates de méthyle) neutralisés.

6. Système thérapeutique en forme de film selon l'une quelconque des revendications précédentes, **caractérisé en ce que** la couche dorsale ou au moins l'une des différentes couches de la couche dorsale contient un ou plusieurs adjuvants, de préférence choisis dans le groupe des plastifiants, des agents de renforcement de la pénétration, des promoteurs de solubilisation, des colorants, des pigments et des agents de formation de matrice.

7. Système thérapeutique en forme de film selon l'une quelconque des revendications précédentes, **caractérisé en ce que** des couches adjacentes contiennent un ou plusieurs polymères de base identiques ou chimiquement apparentés, de préférence du groupe des polyacrylates.

8. Système thérapeutique en forme de film selon l'une quelconque des revendications précédentes, **caractérisé en ce qu'**il est constitué de 2 à 6 couches, de préférence de 2 à 4 couches.

9. Système thérapeutique en forme de film selon l'une quelconque des revendications précédentes, **caractérisé en ce que** la couche dorsale ou la couche extérieure qui est à l'opposé de la couche muco-adhésive forme une couche limite dans laquelle la perméation de l'eau et la diffusion de la substance active est diminuée par rapport à l'autre/aux autres couche(s) du système.

10. Système thérapeutique en forme de film selon l'une quelconque des revendications précédentes, **caractérisé en ce qu'**il est constitué par au moins trois couches et comprend une couche muco-adhésive (2), au moins une couche centrale formant le réservoir (3a) et une couche dorsale extérieure (3b), la dernière couche mentionnée formant une couche limite dans laquelle la perméation de l'eau et la diffusion de la substance active est diminuée par rapport à l'autre/aux autres couche(s) du système.

11. Système thérapeutique en forme de film selon la revendication 9 ou 10, **caractérisé en ce que** la couche limite mentionnée contient des additifs qui diminuent ou bloquent la diffusion de la substance active, en particulier des additifs du groupe des pigments et des polymères ralentissant la diffusion.

12. Système thérapeutique en forme de film selon l'une quelconque des revendications 9 à 11, **caractérisé en ce que** la/les couches de réservoir contient/contiennent un ou plusieurs additifs qui augmentent l'aptitude au gonflement et l'hydratation de la matrice formant le réservoir, ces additifs étant de préférence choisis dans le groupe des substances hydrophiles, liant l'eau, en préférant en particulier les polyalcools et les agents tensioactifs polymères présentant une valeur BHL ≥ 10.

13. Système thérapeutique en forme de film selon l'une quelconque des revendications précédentes, **caractérisé en ce que** la/les substance(s) active(s) se trouve(nt) sous forme dissoute, de suspension ou émulsionnée.

14. Système thérapeutique en forme de film selon l'une quelconque des revendications précédentes, **caractérisé en ce que** deux couches ou plus contiennent la même substance active, de préférence en des concentrations différentes, en formant un gradient de concentration.

15. Système thérapeutique en forme de film selon la revendication 14, **caractérisé en ce que** les couches individuelles contiennent des additifs grâce auxquels la solubilité et le coefficient de diffusion de la substance active est modifié dans la couche respective.
